(19) Europäisches Patentamt
European Patent Office
Office européen des brevets

(11) Veröffentlichungsnummer : **0 029 171**
**B1**

# (12) EUROPÄISCHE PATENTSCHRIFT

(45) Veröffentlichungstag der Patentschrift : **21.03.84**

(21) Anmeldenummer : **80106797.6**

(22) Anmeldetag : **05.11.80**

(51) Int. Cl.³ : **C 07 D261/12**, C 07 D261/20, C 07 D263/38, C 07 D263/40, C 07 D263/42, C 07 D263/52, C 07 D263/58, C 07 D271/04, C 07 D271/06, C 07 D271/08, C 07 D271/10

## (54) Azolyloxy-carbonsäure-N-oxy-amide, Verfahren und Zwischenprodukte zu ihrer Herstellung, ihre Verwendung, sie enthaltende herbizide Mittel und deren Herstellung.

(30) Priorität : **17.11.79 DE 2946524**

(43) Veröffentlichungstag der Anmeldung : **27.05.81 Patentblatt 81/21**

(45) Bekanntmachung des Hinweises auf die Patenterteilung : **21.03.84 Patentblatt 84/12**

(84) Benannte Vertragsstaaten : **BE CH DE FR GB IT LI NL**

(56) Entgegenhaltungen :
**DD-A- 102 702**
**DD-A- 105 230**
**DE-A- 1 445 755**
**DE-A- 1 925 954**
**DE-A- 2 050 346**
**DE-A- 2 058 877**
**DE-A- 2 129 012**
**DE-A- 2 361 613**
**DE-A- 2 535 147**
**DE-A- 2 640 652**
**Chemie d. Pflanzenschutz u. Schädlingsbekämpf. mittel, Berlin 1977, S. 180 u. 511**
**Methodicum Chimicum, Bd. 6, Stuttgart 1974, S. 71-72**

(73) Patentinhaber : **BAYER AG**
**Konzernverwaltung RP Patentabteilung**
**D-5090 Leverkusen 1 Bayerwerk (DE)**

(72) Erfinder : **Förster, Heinz, Dr.**
**Am Eckbusch 47**
**D-5600 Wuppertal 1 (DE)**
Erfinder : **Maurer, Fritz, Dr.**
**Roeberstrasse 8**
**D-5600 Wuppertal 1 (DE)**
Erfinder : **Mues, Volker, Dr.**
**Gellertweg 13**
**D-5600 Wuppertal 1 (DE)**
Erfinder : **Eue, Ludwig, Dr.**
**Paul-Klee-Strasse 36**
**D-5090 Leverkusen 1 (DE)**
Erfinder : **Schmidt, Robert Rudolf, Dr.**
**Hahnenweg 5**
**D-5000 Koeln 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

Azolyloxy-carbonsäure-N-oxy-amide, Verfahren und Zwischenprodukte zu ihrer Herstellung, ihre Verwendung, sie enthaltende herbizide Mittel und deren Herstellung

Es ist bereits bekannt geworden, daß bestimmte Phenoxycarbonsäureamide, wie z. B. 2,4-Dichlorphenoxy-essigsäureamid (vgl. FR-PS 1 313 840) oder 4-Chlor-phenoxyessigsäure-N-methoxy-amid oder -N-methoxy-N-methyl-amid (vgl. Chemie der Pflanzenschutz- und Schädlingsbekämpfungsmittel, Berlin 1977, S. 180 oben und S. 511 unten), herbizid wirksam sind.

Die als Herbizide bekannten Phenoxycarbonsäureamide zeigen jedoch bei den üblichen Aufwandmengen nur eine geringe Wirkung gegen Ungräser und können zur Bekämpfung von Unkräutern in verschiedenen dikotylen Kulturen wegen mangelnder Selektivität nicht verwendet werden.

Es wurden nun neue Azolyloxy-essigsäure-N-oxy-amide der Formel

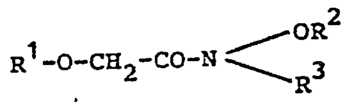

(I)

gefunden, in welcher

$R^1$ für einen der nachstehenden Azolylreste steht

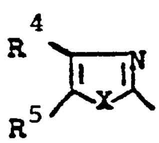
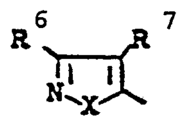
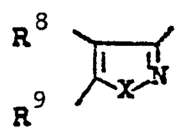
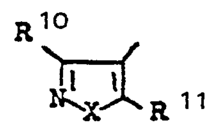
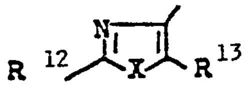
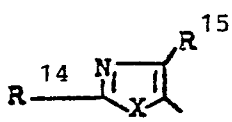
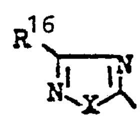
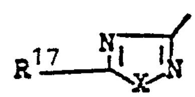
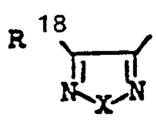
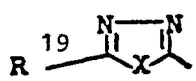
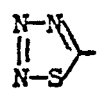
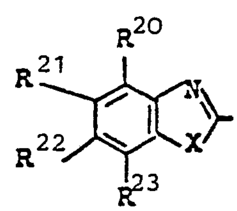

worin

X jeweils für Sauerstoff oder Schwefel steht, die Reste $R^4$ bis $R^{19}$, welche gleich oder verschieden sein können, einzeln für Wasserstoff, Brom, Chlor, Nitro, Cyano, $C_1$-$C_3$-Alkyl-carbonyl, $C_1$-$C_3$-Alkoxycarbonyl, gegebenenfalls durch Fluor, Chlor oder Brom, Methyl, Methoxy, Nitro, Amino und/oder Cyano 1- oder 2fach substituiertes Phenyl, für Phenoxy, Benzyl- oder Phenylthio, für $C_1$-$C_3$-Alkylthio oder $C_1$-$C_3$-Alkoxy, für $C_1$-$C_3$-Alkylsulfinyl oder $C_1$-$C_3$-Alkylsulfonyl, für $C_1$-$C_4$-Alkyl, Trifluormethyl, Cyano-$C_1$-$C_4$-Alkyl, $C_2$-$C_4$-Alkenyl, Benzyloxymethyl, $C_1$-$C_3$-Alkyl-amino, N-$C_1$-$C_3$-Alkyl-N-$C_1$-$C_4$-alkyl-carbonylamino, Phenoxymethyl-benzylthio, $C_1$-$C_3$-Alkyl-carbonyloxy stehen und

die Reste $R^{20}$ bis $R^{23}$, welche gleich oder verschieden sein können, einzeln für Chlor, $C_1$-$C_2$-Alkyl, $C_1$-$C_2$-Alkoxy, Trifluormethyl, Trifluormethoxy oder zusammen für Methylendioxy, Dichlormethylendioxy oder Difluormethylendioxy stehen,

$R^2$ für $C_1$-$C_6$-Alkyl, $C_1$-$C_4$-Alkoxy-ethyl, Allyl oder Propargyl steht und

$R^3$ für $C_1$-$C_6$-Alkyl, $C_1$-$C_4$-Alkoxy-ethyl, Allyl, Propargyl, Cyclopentyl, Cyclohexyl oder Benzyl steht.

Man erhält die neuen Azolyloxy-essigsäure-N-oxy-amide der Formel (I), wenn man Hydroxy-essigsäure-N-oxyamide der Formel

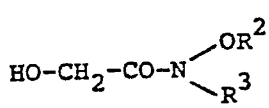

(II)

in welcher

$R^2$ und $R^3$ die oben angegebene Bedeutung haben,

mit Halogenazolen der Formel

$$R^1—Hal \qquad (III)$$

worin

$R^1$ die oben angegebene Bedeutung hat, und

Hal für Chlor, Brom oder Jod steht,

gegebenenfalls in Gegenwart eines Säureakzepters und gegebenenfalls unter Verwendung eines Verdünnungsmittels umsetzt.

Die neuen Azolyloxy-essigsäure-N-oxy-amide der Formel (I) zeichnen sich durch starke herbizide Wirksamkeit aus.

Überraschenderweise zeigen die neuen Azolyloxy-essigsäure-N-oxy-amide eine wesentlich bessere herbizide Wirkung als die aus dem Stand der Technik bekannten Phenoxycarbonsäureamide. Insbesondere überrascht die Tatsache, daß die erfindungsgemäßen Verbindungen bei guter Verträglichkeit gegenüber Nutzpflanzen neben ihrer hohen Wirkung gegen dikotyle Unkräuter auch sehr gute Wirkung gegen Ungräser zeigen, während konstitutionell ähnliche Phenoxy-alkancarbonsäurederivate, wie z. B. 2,4-Dichlorphenoxy-essigsäureamid, nur geringe Wirkung gegen Gramineen aufweisen.

Verwendet man beispielsweise 2,4,5-Trichlor-thiazol und N-Ethoxy-N-ethyl-hydroxyessigsäureamid als Ausgangsstoffe, so kann der Reaktionsablauf nach dem erfindungsgemäßen Verfahren durch folgendes Formelschema skizziert werden :

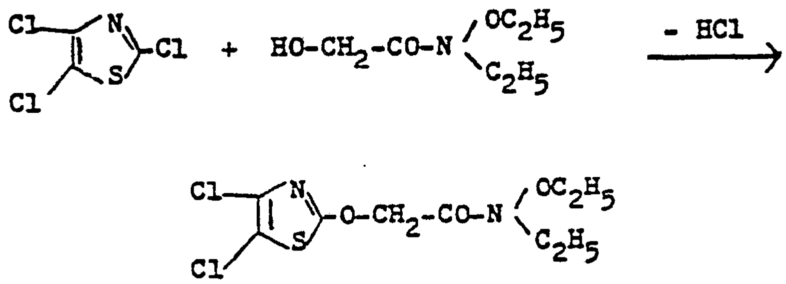


Das Verfahren zur Herstellung der neuen Azolyloxyessigsäureamide wird vorzugsweise unter Verwendung geeigneter Lösungs- oder Verdünnungsmittel durchgeführt. Als solche kommen praktisch alle inerten organischen Solventien infrage. Hierzu gehören insbesondere Alkohole, wie Methanol, Ethanol, n- und iso-Propanol, n-, iso-, sek.- und tert.-Butanol, Ether wie Diethylether, Dibutylether, Tetrahydrofuran und Dioxan, Ketone wie Aceton, Methylethylketon, Methylisopropylketon und Methylisobutylketon, Nitrile wie Acetonitril und Propionsäurenitril, sowie die hochpolaren Lösungsmittel Dimethylformamid, Dimethylsulfoxid, Sulfolan und Hexamethylphosphorsäuretriamid.

Als Säureakzeptoren können praktisch alle üblicherweise verwendbaren Säurebindemittel eingesetzt werden : hierzu gehören insbesondere Alkali- und Erdalkalihydroxide bzw. -oxide wie Natrium- und Kaliumhydroxid sowie Calcium-oxid oderCalcium-hydroxid, Alkali- und Erdalkali-carbonate wie Natrium-, Kalium- und Calciumcarbonat, Alkali-alkoholate wie Natrium-methylat, -ethylat und -tert.-butylat, Kaliummethylat, -ethylat und -tert.-butylat, ferner aliphatische, aromatische oder heterocyclische Amine wie Triethylamin, Dimethylanilin, Dimethylbenzylamin, Pyridin, Diazabicyclooctan und Diazabicycloundecen.

Die Reaktionstemperatur kann innerhalb eines größeren Bereichs variiert werden. Im allgemeinen arbeitet man zwischen − 50 und + 150 °C, vorzugsweise bei − 20 bis + 100 °C.

Das erfindungsgemäße Verfahren wird im allgemeinen bei Normaldruck durchgeführt.

Bei der Durchführung des erfindungsgemäßen Verfahrens setzt man auf 1 Mol Halogenazol der Formel (III) (1,0 bis 1,5 Mol Hydroxy-essigsäureamid der Formel (II) ein. Die Umsetzung wird im allgemeinen in einem geeigneten Verdünnungsmittel durchgeführt und das Reaktionsgemisch wird bei der erforderlichen Temperatur mehrere Stunden gerührt.

Die Isolierung der Produkte erfolgt nach üblichen Methoden : Man destilliert gegebenenfalls einen Teil des Verdünnungsmittels unter vermindertem Druck ab und gießt den Rest der Reaktionsmischung in Wasser.

Soweit die Produkte hierbei kristallin anfallen, werden sie durch Absaugen isoliert. Andernfalls werden die organischen Produkte mit einem mit Wasser nicht mischbaren Lösungsmittel wie z. B. Toluol oder Methylenchlorid extrahiert ; nach Waschen und Trocknen wird dann von der organischen Phase das Lösungsmittel im Vakuum abdestilliert. Die zurückbleibenden Produkte werden durch ihren Schmelzpunkt bzw. ihren Brechungsindex charakterisiert.

Die als Ausgangsstoffe zu verwendenden Hydroxy-essigsäure-N-oxy-amide sind neue Verbindungen.

Als Ausgangsstoffe der Formel (II) seien beispielsweise genannt :

N-Methoxy-N-methyl-, N-Ethoxy-N-methyl-, N-n-Propoxy-N-methyl-, N-iso-Propoxy-N-methyl-, N-Ethoxy-N-ethyl-, N-n-Propoxy-N-ethyl-, N-iso-Propoxy-N-ethyl-, N-n-Propoxy-N-n-propyl-, N-iso-Propoxy-N-isopropyl-, N-iso-Propoxy-N-n-propyl-, N-Methoxy-N-ethyl-, N-Methoxy-N-n-propyl-, N-Methoxy-N-isopropyl-, N-Methoxy-N-n-butyl-, N-Methoxy-N-isobutyl-, N-Methoxy-N-sek.-butyl-, N-Methoxy-N-sek.-hexyl-, N-Ethoxy-N-n-propyl-, N-Ethoxy-N-isopropyl-, N-(2-Ethoxy-ethoxy)-N-methyl-, N-(2-Ethoxy-ethoxy)-N-ethyl-, N-(2-Ethoxy-ethoxy)-N-n-propyl-, N-(2-Ethoxy-ethoxy)-N-isopropyl-, N-(2-Ethoxy-ethoxy)- N-cyclohexyl-, N-Allyloxy-N-allyl-, N-Allyloxy-N-methyl-, N-Allyloxy-N-ethyl-, N-Allyloxy-N-n-propyl, N-Allyloxy-N-isopropyl-, N-Allyloxy-N-n-butyl-, N-Allyloxy-N-iso-butyl-, N-Allyloxy-N-sek.-butyl-, N-Methoxy-N-cyclopentyl-, N-Methoxy-N-cyclohexyl-, N-Methoxy-N-(2-ethoxy-ethyl)-, N-Ethoxy-N-(2-ethoxy-ethyl)-, N-(2-Ethoxy-ethoxy)-N-(2-ethoxy-ethyl)- und N-(2-Ethoxy-ethoxy)-N-sek.-hexyl-α-hydroxy-essigsäureamid.

Die neuen Hydroxyessigsäure-N-oxy-amide der Formel (II) können, wie in nachstehendem Schema skizziert, ausgehend von Chloracetylchlorid (IV) hergestellt werden :

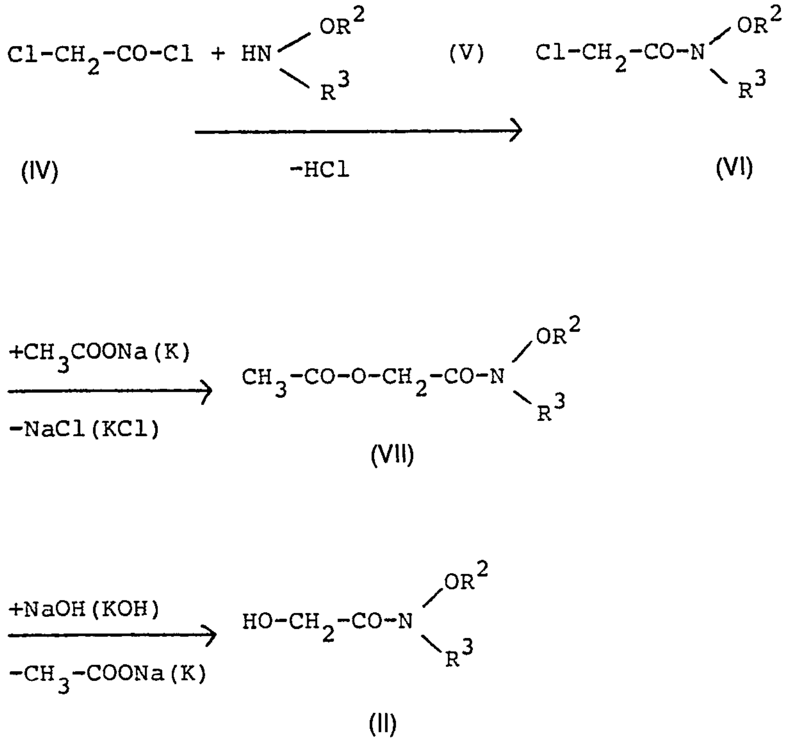


(IV) (VI) (VII) (II)

Hierzu wird zunächst das bekannte Chloracetylchlorid der Formel (IV) mit Hydroxylaminen der Formel (V) — wobei $R^2$ und $R^3$ die oben angegebene.

Bedeutung haben, -gegebenenfalls in Gegenwart eines Säurebindemittels wie z. B. Triethylamin und gegebenenfalls unter Verwendung eines inerten Verdünnungsmittels wie z. B. 1,2-Dichlorethan, bei Temperaturen zwischen − 20 und 100 °C, vorzugsweise zwischen − 10 und 50 °C in die entsprechenden Chloressigsäure-N-oxy-amide der Formel (VI) überführt. Die Aufarbeitung dieser Produkte erfolgt nach üblichen Methoden durch Waschen mit Wasser, Trocknen der organischen Phase und Abdestillieren des Lösungsmittels.

Die Verbindungen der Formel (VI) werden mit Natrium- oder Kalium-acetat, gegebenenfalls unter Verwendung eines Verdünnungsmittels wie z. B. Essigsäure oder Dimethylsulfoxid, bei Temperaturen zwischen 20 und 150 °C, vorzugsweise zwischen 50 und 120 °C, zu den entsprechenden Acetoxy-essigsäure-N-oxy-amiden der Formel (VII) umgesetzt. Soweit die Produkte hierbei kristallin anfallen, werden sie durch Absaugen isoliert. Andernfalls erfolgt die Aufarbeitung nach üblichen Methoden, beispielsweise durch Abdestillieren des Lösungsmittels im Vakuum, Aufnehmen des Rückstandes in Methylenchlorid, Waschen mit Wasser und Abdestillieren des Lösungsmittels.

Durch Umsetzung mit wässrig-alkoholischer Natronlauge oder Kalilauge bei Temperaturen zwischen 0 und 100 °C, vorzugsweise zwischen 10 und 50 °C, können die Verbindungen der Formel (VII) zu den Verbindungen der Formel (II) entacyliert werden. Zur Isolierung der Produkte werden die Lösungsmittel im Vakuum abdestilliert, der Rückstand mit einem organischen Lösungsmittel, wie z. B. Methylenchlorid oder Essigester extrahiert, die Lösung getrocknet und das Lösungsmittel abdestilliert.

Die Hydroxyessigsäure-N-oxy-amide (II) können auch, wie in folgendem Schema skizziert, aus Hydroxyessigsäure (Glykolsäure) (VIII) hergestellt werden :

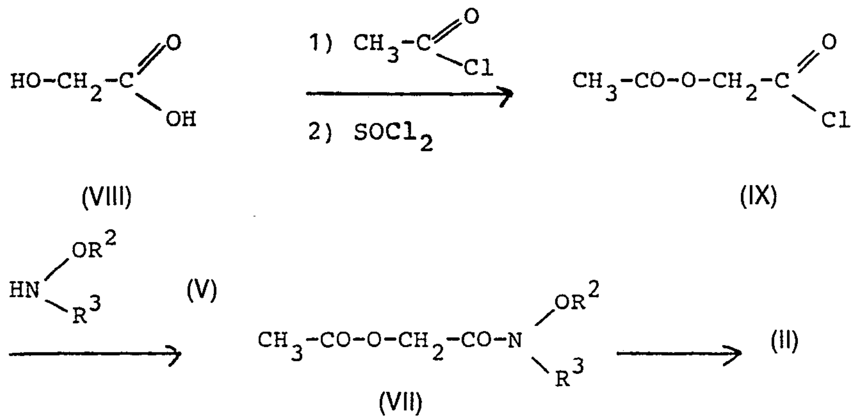


(VIII) (IX) (V) (VII) (II)

Dazu wird zunächst die Hydroxyessigsäure (= Glykolsäure) der Formel (VIII) mit Acylierungsmitteln, wie z. B. Acetylchlorid, bei Temperaturen zwischen 0 und 50 °C, vorzugsweise zwischen 20 und 30 °C, umgesetzt und anschließend das resultierende Reaktionsgemisch mit Chlorierungsmitteln, wie z. B. Thionylchlorid, bei Temperaturen zwischen 20 und 100 °C, vorzugsweise zwischen 40 und 90 °C, behandelt.

Das hierbei erhaltene Acetoxyessigsäurechlorid der Formel (IX), welches durch Destillation gereinigt wird, kann auf konventionelle Weise durch Umsetzung mit den entsprechenden Hydroxylaminen der Formel (V), gegebenenfalls in Gegenwart inerter Verdünnungsmittel, bei Temperaturen zwischen – 10 und + 50 °C, vorzugsweise zwischen 0 und + 30 °C, in die Acetoxyessigsäure-N-oxy-amide der Formel (VII) überführt werden. Diese werden wie oben angegeben zu den Verbindungen der Formel (II) entacyliert.

Die Zwischenprodukte der Formeln (V), (VI) und (VII) sind, soweit darin wenigstens einer der Reste $R^2$ und $R^3$ für Alkenyl- Alkinyl, Cycloalkyl oder Alkoxyalkyl steht, neue Verbindungen.

Man erhält die neuen Hydroxylamine der Formel (V), indem man N-Alkoxycarbonyl-hydroxylamine der Formel

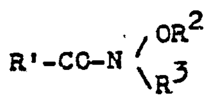

(X)

in welcher

$R^2$ und $R^3$ die oben angegebenen Bedeutungen haben und

R′ für $C_1$-$C_4$-Alkoxy steht,

mit wässrig-alkoholischen Alkalilaugen, wie z. B. mit wässrig-ethanolischer Natronlauge, bei Temperaturen zwischen 10 und 100 °C umsetzt.

Zur Aufarbeitung wird mit Wasser verdünnt, mit einem mit Wasser nicht mischbaren organischen Lösungsmittel, wie z. B. Methylenchlorid, extrahiert ; die Extrakte werden getrocknet, filtriert und destillativ aufgearbeitet.

Die Zwischenprodukte (X) können nach literaturbekannten Verfahren hergestellt werden (vgl. z. B. J. Amer. Chem. Soc. *66* (1944), S. 1931 ; vgl. auch Herstellungsbeispiele).

Als Beispiele für die neuen Ausgangsverbindungen der Formeln (V), (X), (VI) bzw. (VII) seien genannt : N-(2-Ethoxy-ethoxy)-N-isopropyl-amin, -ethoxycarbonylamin, -chloressigsäureamid und -acetoxyessigsäureamid ; N-Allyloxy-N-sek.-butyl-amin, -ethoxycarbonylamin, -chloressigsäureamid und -acetoxyessigsäureamid ; N-(2-Ethoxy-ethoxy)-N-cyclohexyl-amin, -ethoxycarbonylamin, -chloressigsäureamid und -acetoxyessigsäureamid ; N-Methoxy-N-cyclohexyl-amin, ethoxycarbonylamin, -chloressigsäureamid und -acetoxyessigsäureamid ; N-(2-Ethoxy-ethoxy)-N-methyl-amin, ethoxycarbonylamin, -chloressigsäureamid und -acetoxyessigsäureamid ; N-Ethoxy-N-(2-ethoxy-ethyl)-amin, -ethoxy-carbonylamin, -chloressigsäureamid und -acetoxyessigsäureamid ; N-(2-Ethoxy-ethoxy)-N-(2-ethoxy-ethyl)-amin, -ethoxycarbonylamin, -chloressigsäureamid und -acetoxyessigsäureamid ; N-Methoxy-N-allyl-amin, -ethoxycarbonylamin, -chloressigsäureamid und -acetoxyessigsäureamid ; N-(2-Ethoxy-ethoxy)-N-sek.-hexylamin, -ethoxycarbonylamin, -chloressigsäureamid und -acetoxyessigsäureamid ; N-(2-Ethoxy-ethoxy)-N-isopropyl-amin, -ethoxycarbonyl-amin, -chloressigsäureamid und -acetoxyessigsäureamid.

Die weiter als Ausgangsstoffe zu verwendenden Halogenazole sind durch Formel (III) definiert. In dieser Formel steht Hal vorzugsweise für Chlor oder Brom.

Als Ausgangsstoffe der Formel (III) seien beispielsweise genannt :

2-Chlor- und 2-Brom-oxazol und -thiazol, 2,4-Dichlor-, 2,5-Dichlor- und 2,4,5-Trichlor-oxazol und -thiazol, 4-Methyl-, 5-Methyl-, 4-tert.-Butyl-, 4,5-Dimethyl-, 4-Methyl-5-chlor-, 5-Methyl-4-chlor-, 4-Methyl-5-methoxycarbonyl-, 4-Methyl-5-ethoxycarbonyl-, 4-Methyl-5-isopropoxycarbonyl-, 4-Methyl-5-acetyl-, 5-Phenyl-, 4,5-Diphenyl-, 4-Chlor-5-phenyl-, 4-Chlor-5-(3,4-dichlor-phenyl)- und 4-Methyl-5-phenylthio- -2-chloroxazol, -2-brom-oxazol, -2-chlor-thiazol und -2-bromthiazol ; 3-tert.-Butyl-4-cyano-, 3-But-3-en-1-yl-, 3,4-Bis-ethoxycarbonyl-, 3-Phenyl-, 3-Ethyl-4-phenyl-5-chlor-isoxazol, -5-chlor-isothiazol, -5-brom-isoxazol und -5-brom-isothiazol ; 3,5-Bis-ethoxycarbonyl-4-chlor- und 3,5-Bis-ethoxycarbonyl-4-brom-isoxazol und -isothiazol ; 3,5-Dichlor-1,2,4-oxadiazol, 3-Methyl-, 3-Ethyl-, 3-n-Propyl-, 3-iso-Propyl-, 3-tert.-Butyl-, 3-Trifluormethyl-, 3-Trichlormethyl-, 3-Methylthio-, 3-Methylsulfinyl-, 3-Methylsulfonyl-5-chlor-1,2,4-thiadiazol und -5-brom-1,2,4-thiadiazol ; 4-Methyl-, 4-Ethyl-, 4-n-Propyl- und 4-iso-Propyl- -3-chlor-1,2,5-thiadiazol und -3-brom-1,2,5-thiadiazol ; 2-Chlor- und 2-Brom-1,3,4-oxadiazol, 2-Chlor- und 2-Brom-1,3,4-thiadiazol, 5-Methyl-, 5-Ethyl-, 5-n-Propyl-, 5-iso-Propyl-, 5-tert.-Butyl-, 5-Brom-, 5-Methylsulfinyl-, 5-Ethylsulfinyl-, 5-Propylsulfinyl-, 5-Methylsulfonyl-, 5-Ethylsulfonyl-, 5-Propyl-sulfonyl-, 5-Methoxycarbonyl-, 5-Ethoxy-carbonyl-, 5-(1-Cyano-2-methyl-propyl)-, 5-Benzyloxymethyl-, 5-Acetylamino-, 5-Nitro-, 5-Propylthio-, 5-Trifluormethyl-, 5-Trichlormethyl-, 5-Methylamino- und 5-(N-Methyl-N-tert.-butylcarbonylamino)- -2-chlor-1,3,4-oxadiazol, -2-brom-1,3,4-oxadiazol, -2-chlor-1,3,4-thiadiazol und -2-brom-1,3,4-thiadiazol ; 2-Chlor- und 2-Brom-benzoxazol, 2-Chlor- und 2-Brom-benzthiazol, 5-Methyl-2-chlor-benzoxazol, 2-Chlor-6-ethoxy-benzthiazol, 2,5-Dichlor-benzoxazol, 2-Chlor-6-trifluormethyl-benzthiazol, 2-Chlor-5-trifluormethyloxy-benzthiazol, 2-Chlor-5,6-difluormethylendioxy-benzthiazol, 2,4,6,7-Tetrachlorbenzthiazol.

Halogenazole der Formel (III) sind bekannt (vergleiche Elderfield, Heterocyclic Compounds Vol. *5* (1957), S. 298 und S. 452 ; Vol. *7* (1961), S. 463 und S. 541 ; Weissberger, The Chemistry of Heterocyclic Compounds, (a) « Five-Membered Heterocyclic Compounds with Nitrogen and Sulfur or Nitrogen, Sulfur and Oxygen » (1952), S. 35 und S. 81, (b) « Five and Six-Membered Compounds with Nitrogen and Oxygen » (1962), S. 5, S. 245 und S. 263 ; Advances in Heterocyclic Chemistry, Vol. *5* (1965) S. 119 ; Vol. *7* (1966), S. 183 ; Vol. *17* (1974), S. 99 und Vol. *20* (1976), S. 65 ; Synthesis *1978*, 803 ; Tetrahedron Letters *1968*, 829 ; Chem. Ber. *89* (1956), 1534 ; *90* (1957), 182 ; *92* (1959), 1928 ; J. Org. Chem. *27* (1962), 2589 ; DE-OS' 1 670 706, 1 164 413 und 2 213 865).

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden :

Dikotyle Unkräuter der Gattungen : Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen : Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen : Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen : Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere neben einer sehr guten Wirkung gegen grasartige Unkräuter auch eine gute herbizide Wirkung bei breitblättigten Unkräutern. Ein selektiver Einsatz der erfindungsgemäßen Wirkstoffe ist in verschiedenen Kulturen möglich, z. B. in Sojabohnen, Baumwolle, Mais und Rüben.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch

organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und antionische Emulgatoren, wie Polyoxyethylen-Fettsäure-Ester, Polyoxyethylen-Fettalkohol-Ether, z. B. Alkylaryl-polyglykolether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-, Metallphthalocyanin-farbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen oder Stäuben.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,01 und 10 kg Wirkstoff pro ha, vorzugsweise zwischen 0,05 und 8 kg/ha.

Die nachfolgenden Beispiele dienen zur Erläuterung der Erfindung. Die erfindungsgemäßen Wirkstoffe der Formel (I) sowie die Zwischenprodukte der Formeln (II), (VI) und (VII) könnten jeweils auch als Hydroxamsäurederivate bezeichnet werden.

## Herstellungsbeispiele

### Beispiel 1

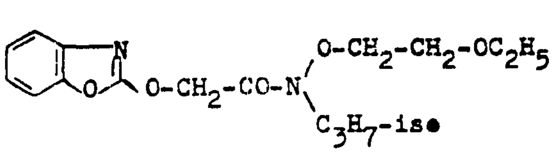

Zu einer Lösung von 6,2 g (0,11 Mol) Kaliumhydroxid in 200 ml Isopropanol tropft man bei 0-5 °C erst 20,5 g (0,1 Mol) Hydroxyessigsäure -N-isopropyl-N-(2-ethoxyethoxy)- amid und dann 15,4 g (0,1 Mol) 2-Chlorbenzoxazol zu und rührt das Gemisch anschließend 6 Stunden ohne Kühlung nach. Dann destilliert man das Lösungsmittel im Vakuum ab, löst den Rückstand in 200 ml Toluol und schüttelt einmal mit 100 ml Wasser aus. Die organische Phase wird über Natriumsulfat getrocknet und im Vakuum eingedampft. Der Rückstand wird bei 60 °C im Hochvakuum andestilliert. Man erhält auf diese Weise 30,3 g (94 % der Theorie) Benzoxazol-2-yl-oxyessigsäure-N-isopropyl-N-(2-ethoxy-ethoxy)-amid in Form eines hellbraunen Öles mit dem Brechungsindex $n_D^{22}$ : 1,498 6.

In analoger Weise können die folgenden Verbindungen der Formel

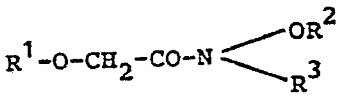

(i)

hergestellt werden :

| Beispiel Nr. | $R^1$ | $R^2$ | $R^3$ | Physikal. Daten (Brechungsindex; Schmelzpunkt °C) |
|---|---|---|---|---|
| 2 | 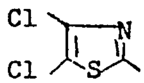 | $-CH_3$ | $-CH_3$ | 89 |
| 3 | 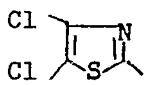 | $-CH_2-CH_2-OC_2H_5$ | $C_3H_7$-iso | $n_D^{22}$:1,5092 |
| 4 | 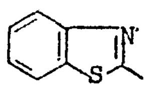 | $C_3H_7$-n | $C_3H_7$-n | 49 |
| 5 | 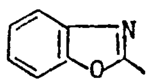 | $C_3H_7$-n | $C_3H_7$-n | 39 |
| 6 | 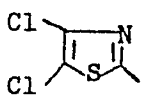 | $C_3H_7$-n | $C_3H_7$-n | $n_D^{21}$:1,5131 |
| 7 | 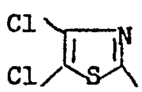 | $C_3H_7$-iso | $C_3H_7$-iso | $n_D^{21}$:1,5122 |
| 8 | 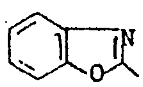 | $C_3H_7$-iso | $C_3H_7$-iso | 44 |
| 9 | 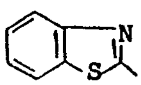 | $C_3H_7$-iso | $C_3H_7$-iso | $n_D^{21}$:1,5491 |
| 10 | 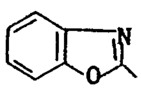 | $-CH_2-CH=CH_2$ | $-CH_2-CH=CH_2$ | 49 |
| 11 | 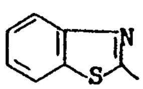 | $-CH_2-CH=CH_2$ | $C_4H_9$-sek. | $n_D^{22}$:1,5582 |
| 12 | 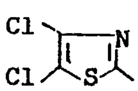 | $-CH_2-CH=CH_2$ | $C_4H_9$-sek. | $n_D^{22}$:1,5167 |
| 13 | 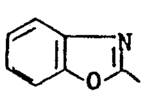 | $-CH_2-CH=CH_2$ | $C_4H_9$-sek. | $n_D^{22}$:1,5219 |

(Fortsetzung)

| Bei-spiel Nr. | $R^1$ | $R^2$ | $R^3$ | Physikal. Daten (Brechungsindex; Schmelzpunkt °C) |
|---|---|---|---|---|
| 14 | 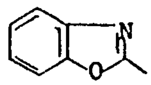 | $-CH_3$ | $C_4H_9$-sek. | 48 |
| 15 | 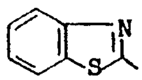 | $CH_3$ | $C_4H_9$-sek. | 52 |
| 16 | 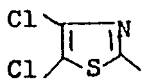 | $-CH_3$ | $C_4H_9$-sek. | $n_D^{22}$:1,5192 |
| 17 | 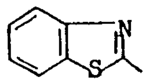 | $-CH_2-CH_2-OC_2H_5$ | $C_3H_7$-iso | $n_D^{20}$:1,5397 |
| 18 | 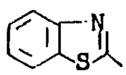 | $-CH_3$ | 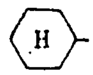 | 78 |
| 19 | 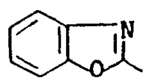 | $-CH_2-CH_2-OC_2H_5$ | 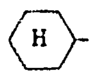 | $n_D^{25}$:1,5138 |
| 20 | 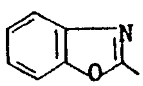 | $-CH_3$ | 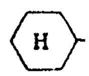 | 53 |
| 21 | 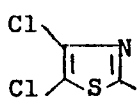 | $-CH_3$ | 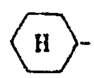 | 78 |
| 22 | 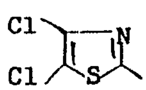 | $-CH_2-CH_2-OC_2H_5$ | 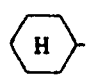 | $n_D^{22}$:1,5231 |
| 23 | 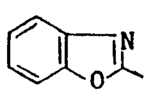 | $-CH_3$ | $-CH_3$ | 107 |
| 24 | 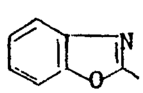 | $-CH_2-CH_2-OC_2H_5$ | $-CH_3$ | $n_D^{22}$:1,5149 |
| 25 | 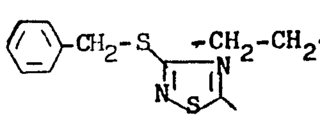 | $-CH_2-CH_2-OC_2H_5$ | $C_3H_7$-iso | $n_D^{22}$:1,4538 |
| 26 | 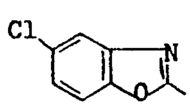 | $-CH_2-CH_2-OC_2H_5$ | $C_3H_7$-iso | $n_D^{22}$:1,5092 |
| 27 | 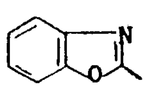 | $C_2H_5$ | $-CH_2-CH_2-OC_2H_5$ | $n_D^{22}$:1,511 |

(Fortsetzung)

| Bei-spiel Nr. | $R^1$ | $R^2$ | $R^3$ | Physikal.Daten (Brechungsindex; Schmelzpunkt °C) |
|---|---|---|---|---|
| 28 | 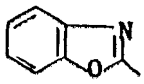 | $-CH_2-CH_2-OC_2H_5$ | $-CH_2-CH_2-OC_2H_5$ | $n_D^{21}$: 1,4988 |
| 29 | 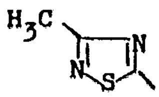 | $-CH_2-CH_2-OC_2H_5$ | $C_3H_7$-iso | 40 |
| 30 | 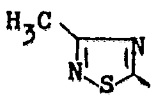 | $-CH_3$ | $C_4H_9$-sek. | $n_D^{21}$: 1,4671 |
| 31 | 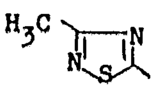 | $C_3H_7$-iso | $C_3H_7$-iso | $n_D^{21}$: 1,4978 |
| 32 | 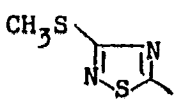 | $-CH_2-CH_2-OC_2H_5$ | $C_3H_7$-iso | $n_D^{20}$: 1,5321 |
| 33 | 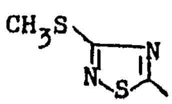 | $C_3H_7$-iso | $C_3H_7$-iso | $n_D^{20}$: 1,4705 |
| 34 | 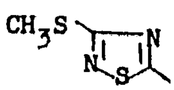 | $-CH_3$ | $C_4H_9$-sek. | $n_D^{20}$: 1,4895 |
| 35 | 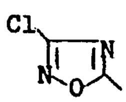 | $-CH_3$ | $C_4H_9$-sek. | $n_D^{20}$: 1,4291 |
| 36 | 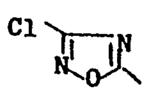 | $C_3H_7$-iso | $C_3H_7$-iso | $n_D^{23}$: 1,4651 |
| 37 | 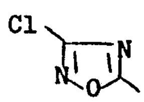 | $-CH_2-CH_2-OC_2H_5$ | $C_3H_7$-iso | $n_D^{22}$: 1,4981 |
| 38 | 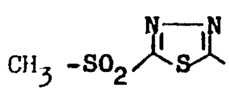 | $-CH_3$ | $C_4H_9$-sek. | 88° |
| 39 | 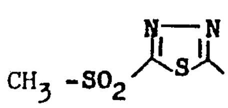 | $-CH_2-CH_2-OC_2H_5$ | $C_3H_7$-iso | 57° |
| 40 | 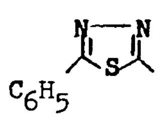 | $C_3H_7$-iso | $C_3H_7$-iso | 102° |

(Fortsetzung)

| Beispiel Nr. | $R^1$ | $R^2$ | $R^3$ | Physikal. Daten (Brechungsindex; Schmelzpunkt °C) |
|---|---|---|---|---|
| 41 | 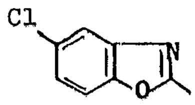 | $C_3H_7$-iso | $C_3H_7$-iso | 83° |
| 42 | 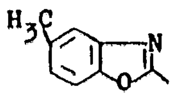 | $-CH_3-CH_2-$ | $C_3H_7$-iso | 51° |
| 43 | 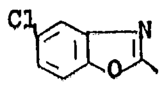 | $-CH_3$ | $C_4H_9$-sek. | Fp:71° |
| 44 | 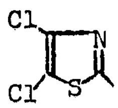 | $-CH_2-CH_2-OC_2H_5$ | $CH_3$<br>CH<br>$C_4H_9$-tert. | $n_D^{20}$:1,5062 |
| 45 | 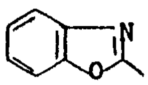 | $-CH_2-CH_2-OC_2H_5$ | $CH_3$<br>-CH<br>$C_4H_9$-tert. | $n_D^{20}$:1,5040 |
| 46 | 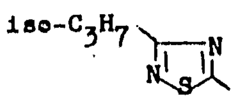 | $C_3H_7$-iso | $C_3H_7$-iso | $n_D^{25}$:1,4861 |
| 47 | 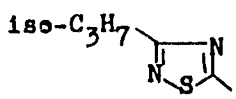 | $-CH_2-CH_2-OC_2H_5$ | $C_3H_7$-iso | $n_D^{22}$:1,4931 |
| 48 | 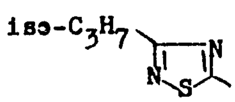 | $-CH_3$ | $C_4H_9$-sek. | $n_D^{20}$:1,4896 |
| 49 | 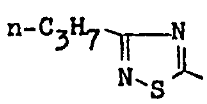 | $-CH_3$ | $C_4H_9$-sek. | $n_D^{22}$:1,4368 |
| 50 | 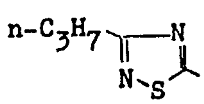 | $-CH_2-CH_2-OC_2H_5$ | $C_3H_7$-iso | $n_D^{22}$:1,5033 |
| 51 | 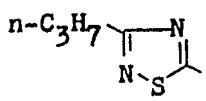 | $C_3H_7$-iso | $C_3H_7$-iso | $n_D^{25}$:1,4825 |
| 52 | 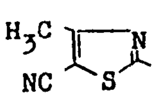 | $C_3H_7$-iso | $C_3H_7$-iso | $n_D^{22}$:1,5055 |

(Fortsetzung)

| Beispiel Nr. | $R^1$ | $R^2$ | $R^3$ | Physikal. Daten (Brechungsindex; Schmelzpunkt °C) |
|---|---|---|---|---|
| 53 | 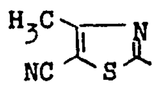 | $-CH_2-CH_2-OC_2H_5$ | $C_3H_7$-iso | $n_D^{22}$:1,5061 |
| 54 | 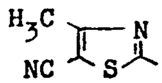 | $-CH_3$ | $C_4H_9$-sek. | Fp.:55° |
| 55 | 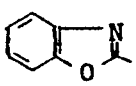 | $-CH_2-CH_2-OCH_3$ | $C_3H_7$-iso | Fp.:32° |

Die als Vorprodukte einzusetzenden Hydroxyessigsäure-N-oxy-amide der Formel (II) können z. B. wie folgt hergestellt werden :

Beispiel 2.1

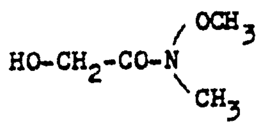

Eine Mischung aus 24,1 g (0,15 Mol) Acetoxyessigsäure-N-methyl-N-methoxy-amid, 100 ml Methanol und 0,2 g Kaliumhydroxid-Pulver wird 2 Stunden unter Rückfluß gekocht. Dann destilliert man das Lösungsmittel im Vakuum ab. Der Rückstand wird im Vakuum destilliert. Man erhält auf diese Weise 16,1 g (90 % der Theorie) Hydroxyessigsäure-N-methyl-N-methoxy-amid als farblose Flüssigkeit mit dem Siedepunkt 47 °C (0,1 mm Hg).

In analoger Weise können die folgenden Verbindungen der Formel

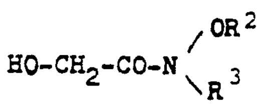 (II)

hergestellt werden :

| Beispiel Nr. | $R^2$ | $R^3$ | Physikal. Daten (Brechungsindex; Siedepunkt °C/Torr) |
|---|---|---|---|
| 2.2 | $-CH_2-CH_2-OC_2H_5$ | $C_3H_7$-iso | $n_D^{24}$:1,4485 |
| 2.3 | $C_3H_7$-n | $C_3H_7$-n | 77-8o/o,1 |
| 2.4 | $C_3H_7$-iso | $C_3H_7$-iso | 63/o,1 |
| 2.5 | $-CH_2-CH=CH_2$ | $-CH_2-CH=CH_2$ | 76-78/o,1 |
| 2.6 | $-CH_2-CH=CH_2$ | $C_4H_9$-sek. | 81/o,1 |

(Fortsetzung)

| Beispiel Nr. | $R^2$ | $R^3$ | Physikal. Daten (Brechungsindex; Siedepunkt °C/Torr) |
|---|---|---|---|
| 2.7 | $-CH_3$ | $C_4H_9$-sek. | 64/0,1 |
| 2.8 | $-CH_3$ | 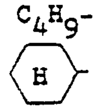 | $n_D^{22}$:1,4849 |
| 2.9 | $-CH_2-CH_2-OC_2H_5$ | 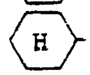 | $n_D^{22}$:1,4748 |
| 2.10 | $-CH_2-CH_2-OC_2H_5$ | $-CH_3$ | 90/0,1 |
| 2.11 | $-CH_2-CH_2-OC_2H_5$ | $-CH_2-CH_2-OC_2H_5$ | $n_D^{22}$:1,4598 |
| 2.12 | $-CH_3$ | $-CH(CH_3)(C_4H_9\text{-tert.})$ | |
| 2.13 | $-CH_2-CH_2-OC_2H_5$ | $-CH(CH_3)(C_4H_9\text{-tert.})$ | $n_D^{20}$:1,4637 |
| 2.14 | $-CH_2-CH_2-OCH_3$ | $C_3H_7$-iso | $n_D^{22}$:1,4587 |
| 2.15 | $C_2H_5$ | $-CH_2-CH_2-OC_2H_5$ | $n_D^{22}$:1,4973 |

Die Darstellung der Acetoxyessigsäure-N-oxy-amide der Formel (VII) z. B. auf folgende Weise erfolgen :

Beispiel 3.1

$$CH_3-CO-O-CH_2-CO-N\begin{matrix}/\ OCH_3\\ \backslash\ CH_3\end{matrix}$$

Eine Mischung aus 23 g (0,28 Mol) Natriumacetat, 200 ml Dimethylsulfoxid und 34,4 g (0,25 Mol) Chloressigsäure-N-methyl-N-methoxy-amid (Darstellung s. DE-OS 2 753 182) wird 7 Stunden bei 70 °C gerührt. Dann kühlt man die Mischung auf Raumtemperatur ab, gibt 500 ml Methylenchlorid zu und saugt vom anorganischen Salz ab. Das Filtrat wird im Vakuum eingedampft, der Rückstand destilliert. Man erhält so 30,3 g (75 % der Theorie) Acetoxyessigsäure-N-methyl-N-methoxy-amid als farblose Flüssigkeit mit dem Siedepunkt 70 °C/0,3 mm Hg.

Beispiel 3.2

$$CH_3-CO-O-CH_2-CO-N\begin{matrix}/\ O-CH_2-CH_2-OC_2H_5\\ \backslash\ C_3H_7\text{-iso}\end{matrix}$$

Eine Mischung aus 33,1 g (0,24 Mol) Kaliumcarbonat, 200 ml Acetonitril und 29,4 g (0,2 Mol) N-Isopropyl-O-(2-ethoxyethyl)-hydroxylamin wird mit 27,3 g (0,2 Mol) 2-Acetoxyacetylchlorid versetzt. Die Temperaur steigt dabei bis ca. 30 °C an. Man rührt noch 2 Stunden ohne Kühlung nach, filtriert das Reaktionsgemisch und destilliert das Lösungsmittel im Vakuum ab. Man erhält so 38 g (77 % der Theorie) Acetoxy-essigsäure-N-(2-ethoxy-ethoxy)-N-isopropyl-amid in Form eines farblosen Öles mit dem Siedepunkt 106-107 °C/0,1 mm Hg.

Beispiel 3.3

$$CH_3-CO-O-CH_2-CO-N\begin{matrix}/\ OC_3H_7\text{-n}\\ \backslash\ C_3H_7\text{-n}\end{matrix}$$

Zu einem Gemisch aus 46,8 g (0,4 Mol) O,N-Dipropylhydroxylamin, 250 ml Ethylenglykoldimethylether und 43,7 g (0,52 Mol) Natriumhydrogencarbonat tropft man bei 10-15 °C 54,6 g (0,4 Mol) 2-Acetoxyacetylchlorid und rührt dann 2 Stunden bei Raumtemperatur nach. Dann saugt man vom abgeschiedenen Salz ab, wäscht mit Ethylenglykoldimethylether nach und dampft dann das Filtrat im Vakuum ein. Zurück bleiben 80 g (92 % der Theorie) Acetoxyessigsäure-N-n-propoxy-N-n-propyl-amid als farblose Flüssigkeit mit dem Brechungsindex $n_D^{21}$ : 1,443 8.

Analog einem der Beispiele 3.1 bis 3.3 können die folgenden Verbindungen der Formel

$$CH_3-CO-O-CH_2-CO-N\begin{matrix} / OR^2 \\ \backslash R^3 \end{matrix}$$

hergestellt werden :

| Beispiel Nr. | $R^2$ | $R^3$ | Brechungsindex; |
|---|---|---|---|
| 3.4 | $C_3H_7$-iso | $C_3H_7$-iso | $n_D^{22}$:1,4482 |
| 3.5 | $CH_2-CH=CH_2$ | $-CH_2-CH=CH_2$ | $n_D^{23}$:1,4642 |
| 3.6 | $CH_2-CH=CH_2$ | $C_4H_9$-sek. | $n_D^{21}$:1,4573 |
| 3.7 | $CH_3$ | $C_4H_9$-sek. | $n_D^{21}$:1,4428 |
| 3.8 | $CH_3$ | 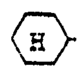 | $n_D^{18}$:1,4668 |
| 3.9 | $CH_2-CH_2-OC_2H_5$ | 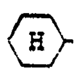 | $n_D^{18}$:1,4646 |
| 3.10 | $CH_2-CH_2-OC_2H_5$ | $-CH_3$ | $n_D^{20}$:1,4487 |
| 3.11 | $CH_2-CH_2-OC_2H_5$ | $-CH_2-CH_2-OC_2H_5$ | $n_D^{20}$:1,4385 |
| 3.12 | $CH_3$ | $-CH\begin{matrix} / CH_3 \\ \backslash C_4H_9\text{-tert.} \end{matrix}$ | $n_D^{22}$:1,4233 |
| 3.13 | $CH_2-CH_2-OC_2H_5$ | $-CH\begin{matrix} / CH_3 \\ \backslash C_4H_9\text{-tert.} \end{matrix}$ | $n_D^{20}$:1,4405 |
| 3.14 | $-CH_2-CH_2-OCH_3$ | $C_3H_7$-iso | $n_D^{20}$:1,4221 |
| 3.15 | $C_2H_5$ | $-CH_2-CH_2-OC_2H_5$ | $n_D^{20}$:1,4831 |

Die z. T. noch nicht beschriebenen O,N-Dialkylhydroxylamine können z. B. folgendermaßen hergestellt werden :

Beispiel 4.1

$$H-N\begin{matrix} / O-CH_2-CH=CH_2 \\ \backslash CH_2-CH=CH_2 \end{matrix}$$

Zu einer Mischung aus 250 ml Wasser, 250 ml Ethanol und 150 ml 45 %ige Natronlauge gibt man 92,5 g (0,5 Mol) O,N-Diallyl-N-carbethoxy-hydroxylamin (Darstellung s. Kleinschmidt und Cope J. Amer. Chem. Soc. *66* (1944), S. 1931). Die Lösung wird 3 Stunden unter Rückfluß gekocht, mit 300 ml Wasser versetzt und 3 mal mit je 200 ml Methylenchlorid extrahiert. Die vereinigten organischen Phasen werden über Natriumsulfat getrocknet, dann destilliert man das Lösungsmittel ab. Der Rückstand wird im Vakuum destilliert. Man erhält so 25 g (44 % der Theorie) O,N-Diallylhydroxylamin als farblose Flüssigkeit mit dem Siedepunkt 33 °C (8 mm Hg).

Beispiel 4.2

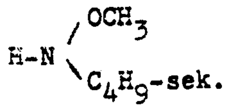

1. Stufe :

N-Phenyl-N'-sek.-butyl-N'-hydroxyharnstoff

Eine Mischung aus 500 ml Ethylenglykoldimethylether, 209,1 g (1,5 Mol) 90 %igem N-sek. -Butylhydroxylamin-Hydrochlorid und 151,2 g (1,8 Mol) Natriumhydrogencarbonat wird 1 Stunde bei Raumtemperatur gerührt und dann bei 0-5 °C mit 178,5 g (1,5 Mol) Phenylisocyanat versetzt.

Man rührt 1 Stunde bei 10 °C nach und saugt dann vom Salz ab und dampft das Filtrat im Vakuum ein. Zurück bleiben 310 g (99 % der Theorie) N-Phenyl-N'-sek.-butyl-N'-hydroxyharnstoff als farbloses Pulver mit dem Schmelzpunkt 107 °C.

2. Stufe :

N-Phenyl-N'-sek.-butyl-N'-methoxyharnstoff

Zu einer Lösung von 60 g (1,5 Mol) Natriumhydroxid und 208 g (1 Mol) des oben beschriebenen Hydroxyharnstoffes in 500 ml Wasser tropft man bei 30 °C 164 g (1,3 Mol) Dimethylsulfat. Man rührt das Gemisch 2 Stunden bei 25-30 °C nach und saugt dann das ausgefallene Produkt ab. Man erhält auf diese Weise 188 g (85 % der Theorie) N-Phenyl-N'-sek.-butyl-N'-methoxy-harnstoff in Form farbloser Kristalle mit dem Schmelzpunkt 53 °C.

3. Stufe :

O-Methyl-N-sek.-butylhydroxylamin

Eine Mischung aus 241 g (2,6 Mol) Anilin und 288,6 g (1,3 Mol) des oben beschriebenen Methoxyharnstoffes wird auf 160 bis 170 °C erhitzt. Dabei destillieren 83 g (62 % der Theorie) O-Methyl-N-sek.-butylhydroxylamin als farblose Flüssigkeit vom Siedepunkt 100 °C/760 mm Hg ab.

Analog einem der Beispiele 4.1 bzw. 4.2 können die folgenden Verbindungen der Formel

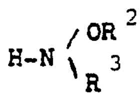

hergestellt werden :

| Beispiel Nr. | $R^2$ | $R^3$ | Siedepunkt (°C/Torr) |
|---|---|---|---|
| 4.3 | $-CH_2-CH_2-OC_2H_5$ | $C_3H_7$-iso | 57/1o |
| 4.4 | $-CH_2-CH=CH_2$ | $C_4H_9$-sek. | 39/1o |
| 4.5 | $-CH_2-CH_2-OC_2H_5$ | 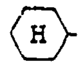 | 63/o,1 |

(Fortsetzung)

| Beispiel Nr. | $R^2$ | $R^3$ | Siedepunkt (°C/Torr) |
|---|---|---|---|
| 4.6 | $-CH_3$ | 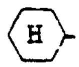 | 63/13 |
| 4.7 | $-CH_2-CH_2-OC_2H_5$ | $-CH_3$ | 49/11 |
| 4.8 | $-C_2H_5$ | $-CH_2-CH_2-OC_2H_5$ | 59/10 |
| 4.9 | $-CH_2-CH_2-OC_2H_5$ | $-CH_2-CH_2-OC_2H_5$ | 97/10 |
| 4.10 | $-CH_3$ | 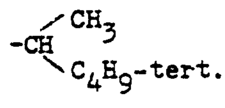 | 88/10 |
| 4.11 | $-CH_2-CH_2-OC_2H_5$ | 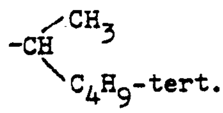 | 45/0,1 |
| 4 12 | $CH_2-CH_2-OCH_3$ | $C_3H_7$-iso | 99/10. |

Beispiel A

Pre-emergence-Test

Lösungsmittel : 5 Gewichtsteile Aceton
Emulgator : 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

Bei diesem Test zeigen z. B. die folgenden Verbindungen der Herstellungsbeispiele eine ausgezeichnete Wirkung :

Nr. 1, 2, 3, 5, 8, 10, 12, 13, 14, 15, 16.

## Ansprüche

1. Azolyloxy-essigsäure-N-oxy-amide der Formel

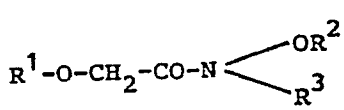

(I)

in welcher
$R^1$ für einen der nachstehenden Azolylreste steht

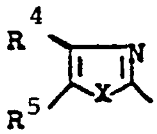 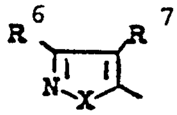 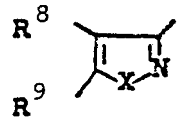 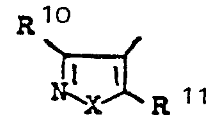

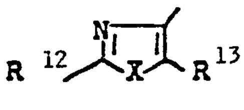 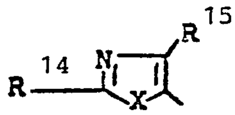 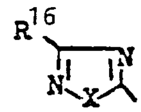 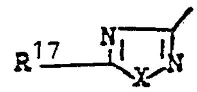

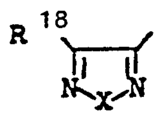 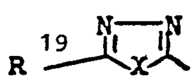 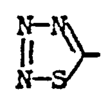 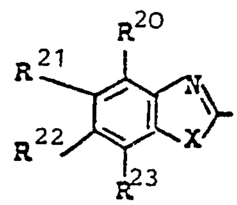

worin

X jeweils für Sauerstoff oder Schwefel steht, die Reste $R^4$ bis $R^{19}$, welche gleich oder verschieden sein können, einzeln für Wasserstoff, Brom, Chlor, Nitro, Cyano, $C_1$-$C_3$-Alkylcarbonyl, $C_1$-$C_3$-Alkoxycarbonyl, gegebenenfalls durch Fluor, Chlor oder Brom, Methyl, Methoxy, Nitro, Amino und/oder Cyano 1- oder 2fach substituiertes Phenyl, für Phenoxy, Benzyl- oder Phenylthio, für $C_1$-$C_3$-Alkylthio oder $C_1$-$C_3$-Alkoxy, für $C_1$-$C_3$-Alkylsulfinyl oder $C_1$-$C_3$-Alkylsulfonyl, für $C_1$-$C_4$-Alkyl, Trifluormethyl, Cyano-$C_1$-$C_4$-Alkyl, $C_2$-$C_4$-Alkenyl, Benzyloxymethyl, $C_1$-$C_3$-Alkyl-amino, N-$C_1$-$C_3$-Alkyl-N-$C_1$-$C_4$-alkyl-carbonylamino, Phenoxymethyl-benzylthio, $C_1$-$C_3$-Alkyl-carbonyloxy stehen und

die Reste $R^{20}$ bis $R^{23}$, welche gleich oder verschieden sein können, einzeln für Chlor, $C_1$-$C_2$-Alkyl, $C_1$-$C_2$-Alkoxy, Trifluormethyl, Trifluormethoxy oder zusammen für Methylendioxy, Dichlormethylendioxy oder Difluormethylendioxy stehen,

$R^2$ für $C_1$-$C_6$-Alkyl, $C_1$-$C_4$-Alkoxy-ethyl, Allyl oder Propargyl steht und

$R^3$ für $C_1$-$C_6$-Alkyl, $C_1$-$C_4$-Alkoxy-ethyl, Allyl, Propargyl, Cyclopentyl, Cyclohexyl oder Benzyl steht.

2. Verfahren zur Herstellung von Azolyloxy-essigsäure-N-oxy-amiden der Formel (I) gemäß Anspruch 1, dadurch gekennzeichnet, daß man Hydroxyessigsäure-N-oxy-amide der Formel

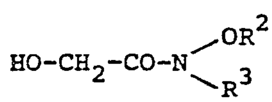 (II)

in welcher

$R^2$ und $R^3$ die in Anspruch 1 angegebene Bedeutung haben, mit Halogenazolen der Formel

$$R^1\text{—Hal} \qquad (III)$$

worin

$R^1$ die in Anspruch 1 angegebene Bedeutung hat und

Hal für Chlor, Brom oder Jod steht, gegebenenfalls in Gegenwart eines Säureakzeptors und gegebenenfalls unter Verwendung eines Verdünnungsmittels umsetzt.

3. Herbizide Mittel, gekennzeichnet, durch einen Gehalt an Azolyloxy-essigsäure-N-oxy-amiden der Formel (I) gemäß Anspruch 1.

4. Verwendung von Azolyloxy-essigsäure-N-oxy-amiden der Formel (I) gemäß Anspruch 1 zur Bekämpfung von Pflanzenwachstum.

5. Verfahren zur Herstellung von herbiziden Mitteln, dadurch gekennzeichnet, daß man Azolyloxy-essigsäure-N-oxy-amide der Formel (I) gemäß Anspruch 1 mit Streckmitteln und/oder oberflächenaktiven Mitteln vermischt.

6. Hydroxyessigsäure-N-oxy-amide der Formel

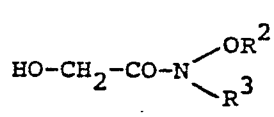 (II)

in welcher $R^2$ und $R^3$ die in Anspruch 1 angegebene Bedeutung haben.

**Claims**

1. Azolyloxy-acetic acid N-oxy-amides of the formula

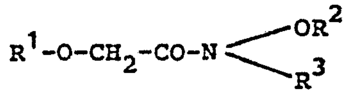

(I)

in which

$R^1$ represents one of the azolyl radicals below

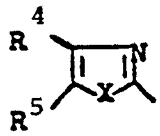 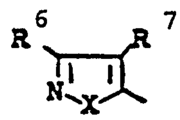 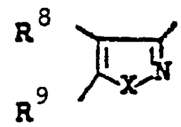 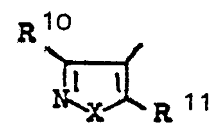

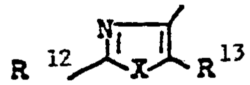 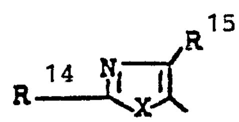 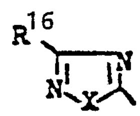 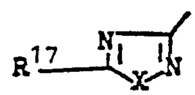

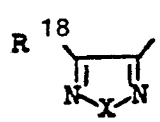 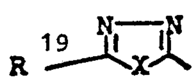 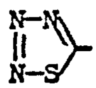 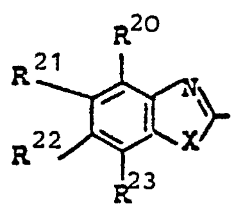

wherein

X in each case represent oxygen or sulphur, the radical $R^4$ to $R^{19}$, which can be identical or different, individually represent hydrogen, bromine, chlorine, nitro, cyano, $C_1$-$C_3$-alkyl-carbonyl, $C_1$-$C_3$-alkoxycarbonyl, phenyl which is optionally monosubstituted or disubstituted by fluorine, chlorine or bromine and methyl, methoxy, nitro, amino and/or cyano, phenoxy, benzylthio or phenylthio, $C_1$-$C_3$-alkylthio or $C_1$-$C_3$-alkoxy, $C_1$-$C_3$-alkylsulphinyl or $C_1$-$C_3$-alkylsulphonyl, $C_1$-$C_4$-alkyl, trifluoromethyl, cyano-$C_1$-$C_4$-alkyl, $C_2$-$C_4$-alkenyl, benzyloxymethyl, $C_1$-$C_3$-alkylamino, N-$C_1$-$C_3$-alkyl-N-$C_1$-$C_4$-alkyl-carbonyl-amino, phenoxymethyl-benzylthio or $C_1$-$C_3$-alkyl-carbonyloxy, and

the radicals $R^{20}$ to $R^{23}$, which can be identical or different, individually represent chlorine, $C_1$-$C_2$-alkyl, $C_1$-$C_2$-alkoxy, trifluoromethyl or trifluoromethoxy, or together represent methylenedioxy, or together represent methylenedioxy, dichloromethylenedioxy or difluoromethylenedioxy,

$R^2$ represents $C_1$-$C_6$-alkyl, $C_1$-$C_4$-alkoxy-ethyl, allyl or propargyl and

$R^3$ represents $C_1$-$C_6$-alkyl, $C_1$-$C_4$-alkoxy-ethyl, allyl propargyl, cyclopentyl, cyclohexyl or benzyl.

2. Process for the preparation of azolyloxy-acetic acid N-oxy-amides of the formula (I) according to Claim 1, characterised in that hydroxy-acetic acid N-oxy-amides of the formula

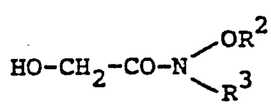

(II)

in which

$R^2$ and $R^3$ have the meaning indicated in Claim 1, are reacted with halogenoazoles of the formula

$R^1$—Hal (III)

wherein

$R^1$ has the meaning indicated in Claim 1 and

Hal represents chlorine, bromine or iodine, if appropriate in the presence of an acid acceptor and if appropriate using a diluent.

3. Herbicidal agents, characterised in that they contain azolyloxy-acetic acid N-oxy-amides of the formula (I) according to Claim 1.

4. Use of azolyoxy-acetic acid N-oxy-amides of the formula (I) according to Claim 1 for combating plant growth.

5. Process for the preparation of herbicidal agents, characterised in that azolyoxy-acetic acid N-oxy-amides of the formula (I) according to Claim 1 are mixed with extenders and/or surface-active agents.

6. Hydroxyacetic acid N-oxy-amides of the formula

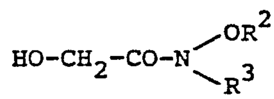

(II)

in which $R^2$ and $R^3$ have the meaning indicated in Claim 1.

## Revendications

1. N-oxyamides d'acides azolyloxy-acétiques de formule

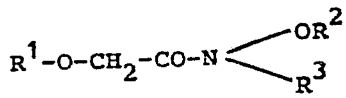

(I)

dans laquelle

$R^1$ représente l'un des restes azolyle suivants :

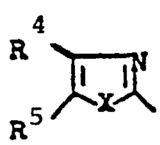
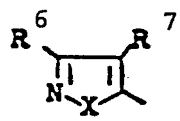
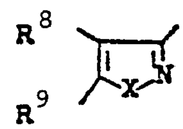
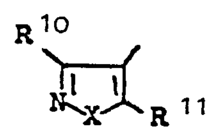
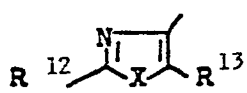
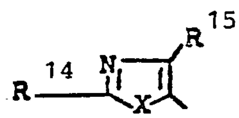
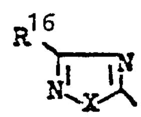
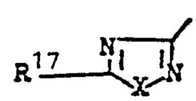
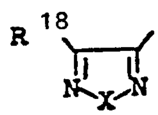
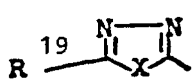
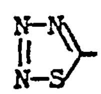
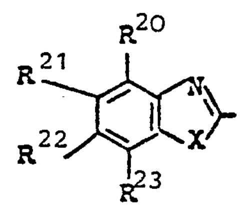

dans lesquels

X désigne dans chaque cas l'oxygène ou le soufre, les restes $R^4$ à $R^{19}$, qui peuvent être égaux ou différents, représentent individuellement l'hydrogène, le brome, le chlore, un groupe nitro, cyano, (alkyle en $C_1$ à $C_3$) carbonyle, (alkoxy en $C_1$ à $C_3$) carbonyle, phényle éventuellement substitué une ou deux fois par du fluor, du chlore ou du brome, un radical méthyle, méthoxy, nitro, amino et/ou cyano, un groupe phénoxy, benzyle ou phénylthio, un groupe alkylthio en $C_1$ à $C_3$ ou alkoxy en $C_1$ à $C_3$, un groupe alkylsulfinyle en $C_1$ à $C_3$ ou un groupe alkylsulfonyle en $C_1$ à $C_3$, un groupe alkyle en $C_1$ à $C_4$, trifluorométhyle, cyanalkyle en $C_1$ à $C_4$, alcényle en $C_2$ à $C_4$, benzyloxyméthyle, alkylamino en $C_1$ à $C_3$, N-(alkyle en $C_1$ à $C_3$)-N-(alkyle en $C_1$ à $C_4$)-carbonylamino, phénoxyméthyl-benzylthio, (alkyle en $C_1$ à $C_3$)-carbonyloxy et

les restes $R^{20}$ à $R^{23}$, qui peuvent être égaux ou différents, représentent, individuellement, du chlore ou un reste alkyle en $C_1$ ou $C_2$, alkoxy en $C_1$ ou $C_2$, trifluorométhyle, trifluorométhoxy ou forment, conjointement, un reste méthylènedioxy, dichlorométhylènedioxy ou difluorométhylènedioxy,

$R^2$ est un groupe alkyle en $C_1$ à $C_6$, (alkoxy en $C_1$ à $C_4$)-éthyle, allyle ou propargyle et

$R^3$ est un groupe alkyle en $C_1$ à $C_6$, (alkoxy en $C_1$ à $C_4$) éthyle, allyle, propargyle, cyclopentyle, cyclohexyle ou benzyle.

2. Procédé de production de N-oxyamides d'acides azolyloxy-acétiques de formule (I) suivant la revendication 1, caractérisé en ce qu'on fait réagir des N-oxyamides d'acide hydroxyacétique de formule

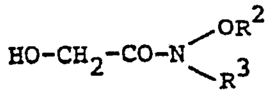

(II)

dans laquelle

$R^2$ et $R^3$ ont la définition indiquée dans la revendication 1, avec des halogénazoles de formule

$R^1$—Hal

(III)

dans laquelle

$R^1$ a la définition indiquée dans la revendication 1 et

Hal désigne du chlore, du brome ou de l'iode, éventuellement en présence d'un accepteur d'acide et en utilisant, le cas échéant, un diluant.

3. Compositions herbicides, caractérisées par une teneur en N-oxyamides d'acides azolyloxy-acétiques de formule (I) suivant la revendication 1.

4. Utilisation de N-oxyamides d'acides azolyloxy-acétiques de formule (I) suivant la revendication 1 pour lutter contre une végétation.

5. Procédé de préparation de compositions herbicides, caractérisé en ce qu'on mélange des N-oxyamides d'acides azolyloxy-acétiques de formule (I) suivant la revendication 1 avec des diluants et/ou des agents tensio-actifs.

6. N-oxyamides d'acide hydroxy-acétique de formule

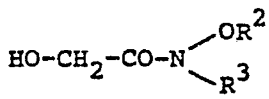

(II)

dans laquelle $R^2$ et $R^3$ ont la définition indiquée dans la revendication 1.